# EUROPEAN PATENT SPECIFICATION

(11) **EP 2 656 818 B1**
(45) Date of publication and mention of the grant of the patent: **25.01.2023**
(21) Application number: 11850312.7
(22) Date of filing: 08.12.2011
(51) Int. Cl.: A61F 2/90, A61L 27/04, A61F 2/915, A61L 31/02, A61L 31/14, A61F 2/89, A61F 2/91, A61F 2/82

(54) **ABSORBABLE BLOOD VESSEL STENT**
ABSORBIERBARER BLUTGEFÄSS-STENT
ENDOPROTHÈSE ABSORBABLE POUR VAISSEAU SANGUIN

(30) Priority: 21.12.2010 CN 201010598542
(43) Date of publication of application: 30.10.2013
(73) Proprietor: Biotyx Medical (Shenzhen) Co., Ltd., Shenzhen, Guangdong 518000 (CN)
(72) Inventor: WANG, Yongsheng, Shenzhen Guangdong 518057 (CN); ZHANG, Deyuan, Shenzhen Guangdong 518057 (CN); XIN, Chaohua, Shenzhen Guangdong 518057 (CN)
(74) Representative: Prinz & Partner mbB
(86) International application number: PCT/CN2011/083683
(87) International publication number: WO 2012/083796

(56) References cited:
- WO-A1-2010/030873
- WO-A1-2010/088676
- WO-A2-02/26109
- WO-A2-2010/102254
- CN-A- 101 102 728
- CN-U- 201 572 353
- CN-U- 201 968 866
- CN-Y- 2 787 169
- US-A1- 2004 249 443
- US-A1- 2008 065 196
- US-B1- 6 287 332

## Description

The invention relates to a medical instrument, and in particular, to an absorbable blood vessel stent for treatment of blood vessel lumen stenosis.

In 1977, the first case of Percutaneous Coronary Intervention (PCI) surgery in the world was accomplished in Switzerland. In this surgery, a balloon was employed to dilate the position of the left anterior descending branch stenosis of the patient, sucessfully. However, the re-stenosis rate of the blood vessel after balloon dilation was very high, over 50%. The main reason was that, after the blood vessel was dilated and the external force was removed, the blood vessel may elastically recoil. In the 1980s, blood vessel stents widely used for coronary artery were invented. There were blood vessel metal stents mainly made of stainless steel, cobalt-chromium alloy or nickel-titanium alloy. After implanted into the human body, the stents may provide durable mechanical support for the blood vessel, thereby preventing the blood vessel from elastically recoiling and reducing the re-stenosis rate of the diseased blood vessel. However, since the stents were permanently implanted into the human body, the stents as foreign substances may cause intimal hyperplasia and quite high re-stenosis rate of the blood vessel.

Since 2003, the application of stents treated with drugs has reduced the blood vessel re-stenosis rate to about 10%. However, the drug stents still existed inside the human body permanently. As foreign objects the stents have great differences with vascular tissues in terms of mechanical properties, which may cause chronic injury of the blood vessel or cause blood vessel medial atrophy and intimal hyperplasia at the late stage. This may also finally cause blood vessel re-stenosis, thereby limiting the further reduction of the blood vessel re-stenosis rate. As for children, the implantation of blood vessel stents with fixed sizes may hinder the gradual increase of the blood vessel, being unable to meet the requirement on the growth of children.

Both of the above intervention treatment technologies for blood vessel stenosis have drawbacks. When dilating the blood vessel by using balloons, although the short-term effect is good, the re-stenosis rate at the late stage is very high because of elastic recoiling of the blood vessel. When implanting metal stents into the blood vessel, the metal stent - including a bare stent or drug stent - may dilate the narrow blood vessel and provide a durable mechanical support, however, the blood vessel intima may be injured during expansion of the metal stents, which may induce blood vessel intimal hyperplasia and lead to re-stenosis. The metal stents further have the drawbacks of thrombosis, coagulation complication, flexibility mismatching, and increase of the re-stenosis occurrence rate at late stage if they permanently remain inside the human body.

In order to solve the above problems, many people began to pay close attention to the blood vessel stent absorbable by the human body in recent years. After a blood vessel stent is implanted into the human body, the ideal situation should be that, at the initial stage, the blood vessel stent provides a sufficient support to the blood vessel, and meanwhile releases drugs to treat the diseased blood vessel. And after the treating function is accomplished, the blood vessel stent is gradually absorbed so as to prevent the re-stenosis. Using different materials, there are two main kinds of absorbable blood vessel stents. One kind is the blood vessel stent made of macromolecular polymer materials, such as polylactic acid; and the other kind is the blood vessel stent made of metal materials, such as magnesium alloy and iron.

Recently, scientists have been doing research on making blood vessel stents, for example, a polylactic acid stent, by degradable macromolecular materials, and some research results have started clinical tests. However, compared with the metal materials, the degradable macromolecular materials have obvious defects in terms of mechanical properties, and so the application thereof is limited. When compared with the metal materials, the macromolecular polymer materials have low mechanical properties and insufficient strength. In order to achieve certain radial support, the wall thickness of the stent must be increased. However, after implanted inside the human body, the stent with a thick wall may hinder the flow of the blood. In addition, the retraction rate of the macromolecular polymer material stents is quite high when expanded by the balloons, and so the expansion ratio of the diameter of the stent after expanded with respect to the diameter of the blood vessel is larger than that of the metal material stents, which may cause greater injury to the blood vessel during the dilation. The X-ray visibility of the macromolecular polymer material stents inside the human body is poor, and so it is difficult to locate and observe the stents during the implantation.

At present, there are mainly two types of metal materials applied to absorbable blood vessel stents: magnesium alloy material and pure iron material. The magnesium alloy material is poor in mechanical properties and fast in corrosion rate. The magnesium alloy material has good bio-compatibility. However, the maximum elongation rate of the magnesium alloy stents is low, which brings great challenge to the structural design of the magnesium alloy stents, and so it is difficult to ensure the good mechanical properties of the stents. Furthermore, the corrosion rate of the magnesium alloy stents is too fast, so a complex material manufacture process must be adopted to control the rate by which the magnesium alloy stents are absorbed by the human body.

Iron is an essential element for the human body, and pure iron has good bio-compatibility and mechanical properties simultaneously. Compared with the polymer stents or magnesium alloy stents having a same wall thickness, the iron stents may provide a sufficient radial support for the diseased blood vessel at the diseased position. However, the corrosion rate of the pure iron is relatively slow. Thus the iron stents of common structure design may be absorbed by the human body after a long time, during which process the diameter of the blood vessel may be restrained. As a result it cannot meet the requirement on the gradual increase of the blood vessel diameter for children.

Therefore, an urgent problem to be solved at present is how to design a blood vessel stent that can accelerate the absorption process of the human body and simultaneously ensure the mechanical properties thereof.

US 2008/0065196A1 discloses a stent having a plurality of serpentine bands, each band having a plurality of struts so as to form alternating peaks and troughs. When in the expanded state, each of the interconnected struts of each serpentine band comprises slits. Each slit is non-linear and formed continuously from a first end to a second end. The slit extends lengthwise over at least a portion of three consecutively connected struts.

The technical problem to be solved is then to provide a blood vessel stent which is used for treating blood vessel stenosis and is absorbable by the human body, so as to solve the problem in the prior art that two properties of the stents are hard to balance. That is to balance the mechanical properties of the absorbable blood vessel stents and to have the blood vessel stent be absorbed as fast as possible.

### Summary of the Invention

According to the present invention, an absorbable blood vessel stent is provided as defined in claim 1. The stent has a proximal end and a distal end, a pattern structure that is tubular and expandable is formed between the proximal end and the distal end, where the pattern structure comprises a plurality of support struts and connection bridges. The connection bridge or support strut comprises a linear, a U-shaped , or an S-shaped segment, and at least one through slot is provided on first and second support struts.

A strut of the stent has at least one neck portion located near the slot, where the neck portion defines a minimum width along the strut.

First and second through slots are provided in the first and second struts parallel to the linear segment of the respective strut. Any one of the through slots and the linear segment form two branches, and the branches have a minimum width along the strut.

The neck portion is formed at an end of the branch, the neck portion has the minimum width along the support strut; and a broad portion is formed at the other end of the branch, where the broad portion has the maximum width on the branch.

The pattern structure of the present invention comprises several annular wavy bands where two adjacent bands are coupled together. The annular wavy bands are interconnected by the connection bridges of the pattern structure. Each annular band comprises a plurality of arched support pieces connected head to tail and a plurality of wave valley segments. Two adjacent arched support pieces are connected by such a wave valley segment. The arched support piece comprises a wave peak segment, a first support strut, and a second support strut. The first support strut and the second support strut are connected by the peak segment.

As a further embodiment of the absorbable blood vessel stent, the first strut and the second strut are symmetric with each other.

As a further embodiment of the absorbable blood vessel stent, the minimum width on the support strut is 0.05 to 0.1 mm.

As a further embodiment of the absorbable blood vessel stent, the blood vessel stent is made of iron or iron alloy material.

### Beneficial Effects

Compared with the prior art, the invention has the following advantages: 1. The absorbable blood vessel stent of the invention with a special structure can become corroded and decompose only within a short time, which reduces the re-stenosis probability of the diseased blood vessel, contributes to continuous growth and expansion after the repair of the diseased blood vessel, and meets requirements of clinical use; 2. The structure of the absorbable blood vessel stent of the invention does not sacrifice the mechanical properties of the blood vessel stent. While promoting the corrosion and disintegration of the blood vessel stent, the blood vessel stent maintains the sufficient radial support force for the diseased blood vessel before disintegration; and 3. The absorbable blood vessel stent of the invention may be made of an iron tube, the wall thickness of the blood vessel stent is not increased compared with a common permanent blood vessel stent, and the stent may be delivered by adopting a balloon catheter which is commonly used for clinical application. Thus the cost of application of absorbable blood vessel stent is reduced, and the clinically applicable scope of the absorbable blood vessel stent is extended.

### Brief Description of the Drawings

Fig. 1 is a schematic diagram of an absorbable blood vessel stent prior to laser engraving molding according to an embodiment of the present invention;
Fig. 2 is a schematic diagram of an absorbable blood vessel stent after laser engraving that is not yet expanded according to an embodiment of the present invention;
Fig. 3 is a plane diagram of an annular wavy band of the blood vessel stent in Fig. 2;
Fig. 4 is a schematic diagram of a first embodiment of an arched support piece of an annular wavy band in Fig. 3;
Fig. 5 is a schematic diagram of a second embodiment of an arched support piece of an annular wavy band in Fig. 3;
Fig. 6 is a schematic diagram of a configuration, not part of the invention, of an arched support piece of an annular wavy band in Fig. 3;
Fig. 7 is a schematic diagram of an embodiment of an arched support piece of an annular wavy band in Fig. 3;
Fig. 8 is a schematic diagram of a configuration, which is not part of the invention, of an arched support piece of an annular wavy band in Fig. 3;
Fig. 9 is a schematic diagram of a configuration, which is not part of the invention, of an arched support piece of an annular wavy band in Fig. 3; and
Fig. 10 is a schematic diagram of a configuration, which is not part of the invention, of an arched support piece of an annular wavy band in Fig. 3.

### Detailed Description of the Embodiments

To make the purposes, technical solutions and advantages of the invention more clear, the invention will be further described in details as below by embodiments with reference to the drawings. It should be understood that the specific embodiments described herein are only used for explaining the invention, but not for limiting the invention.

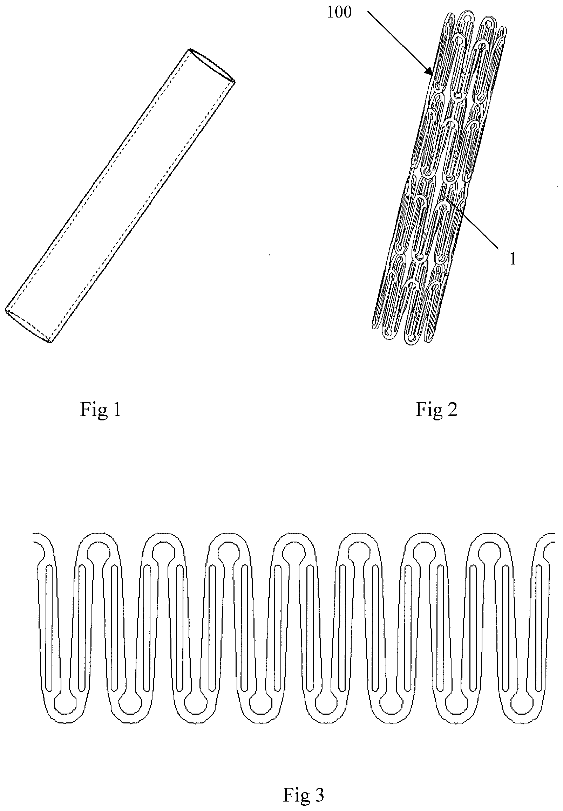

As shown in Fig. 1, the absorbable blood vessel stent in the first embodiment of the invention is a thin iron tube with a diameter of 2mm and a wall thickness of 0.1 mm before laser engraving. Fig. 2 shows a blood vessel stent 100 that is formed after the iron tube is engraved by laser and that is not expanded yet. The blood vessel stent 100 has a proximal end and a distal end, and a expandable pattern structure exists between the proximal end and the distal end. The stent consists of four annular wavy bands in total, where each annular band is formed by eight arched support pieces which are connected head to tail to form a circle. Each annular wavy band is connected to another by linear connection bridges 1. It may be understood that, the blood vessel stent 100 may also consist of two, three, five or more annular bands. Also, there may be more than or less than eight arched support pieces included in each annular band. The connection bridge 1 may also be in another shape, such as a U shape or S shape.

The blood vessel stent 100 may be made of iron alloy or other materials. The pattern structure in a known absorbable blood vessel stent or other blood vessel stents can be used in the absorbable blood vessel stent, as long as it consists of support struts and connection bridges.

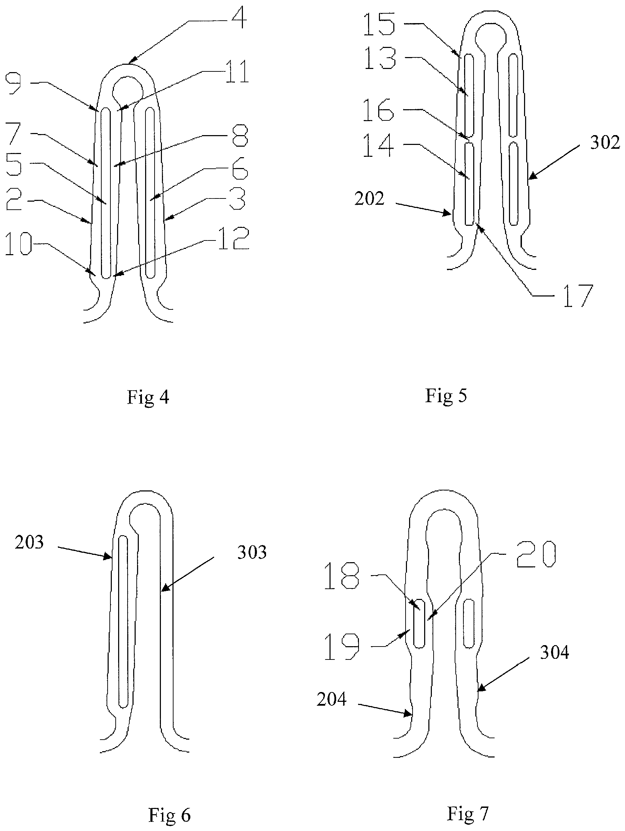

Fig. 3 is a plane diagram of an annular band after the blood vessel stent 100 is slightly expanded radially and Fig. 4 is a first embodiment of an arched support piece of the annular band. The arched support piece comprises a first support strut 2 and a second support strut 3 with an angle to each other, and the first support bar 2 and the second support strut 3 essentially are single linear sections and are connected by the wave peak segment 4 at the top of the arch. In order to make the blood vessel stent have different properties, the first support strut 2 and the second support strut 3 may be symmetric, or may be asymmetric. The support strut may not only be in the shape of single straight line segment shown in Fig. 4, but also be in a curved shape containing several straight segments, for example, a U shape, a S shape, or a combination of these shapes and other shapes. Only the straight segments therein need to be configured in a similar manner.

Adjacent arched support pieces are connected by the wave valley segments at the bottom. The valley segments of one annular wavy band may be connected with a peak segment of the adjacent annular wavy band by the connection bridge 1. The peak segment of a single annular band is closer to the distal end of the stent than the valley segment. The peak segments 4 and valley segments may be in different shapes, and may also be symmetrical structures.

A first through slot 5 is provided in the central portion of the first strut 2, a second through slot 6 is provided in the central portion of the second strut 3. Both ends of the first slot 5 and the second slot 6 are respectively close to positions of the peak segment 4 and the valley segment which are connected with corresponding struts. The first slot 5 divides the middle portion of the first strut 2 into a first branch 7 and a second branch 8. A neck portion 9 is provided at the position where the first branch 7 is close to the peak segment 4. The neck portion 9 is the position with the minimum width on the first branch 7, wherein the minimum width may be in the range from 0.05 to 0.1 mm. A broad portion 10 is arranged at the other end of the first branch 7, and is the position with the maximum width on the first branch 7, wherein the width of the broad portion 10 may be in the range from 0.1 to 0.2 mm. On the first branch 7, the width from the neck portion 9 to the broad portion 10 may change gradually.

Another broad portion 11 is provided at the position opposite to the neck portion 9 on the second branch 8, and the width may be in the range from 0.1 to 0.2 mm, and the broad portion 11 is the position with the maximum width on the second branch 8. Another neck portion 12 with a width between 0.05 and 0.1 mm is provided at the position opposite to the broad portion 10 on the second branch 8, and is the position with the minimum width on the second branch 8. On the second branch 8, the width may change gradually from the neck portion 12 to the broad portion 11.

When the blood vessel stent 100 is expanded and deformed, since the strength of the broad portions 10 and 11 is larger than the strength of the neck portions 9 and 12, the broad portion 11 may protect the neck portion 9 from large deformation, and the broad portion 10 may protect the neck portion 12 from large deformation. After the blood vessel stent 100 is implanted into the body, since the neck portions 9 and 12 are respectively the parts with the minimum widths on the first branch 7 and the second branch 8, the neck portion 9 and the neck portion 12 may break first because of corrosion. This would cause the first support strut 2 to completely break, and the blood vessel stent 100 may come apart at this place in a shorter time. The second support strut 3 has the structure and technical effect similar to the first support strut 2, and breaks from corrosion in the human body after approximately the same time.

During expansion of the stent, the first branch 7 and the second branch 8 located at both sides of the slot 5 may be deformed simultaneously. The broad portion 11 is close to and aligned with the neck portion 9. The width of the broad portion 11 is larger, and so it is hard to be deformed during the expansion process. Therefore, the stress for deforming the first strut 2 is mainly distributed at the broad portion 11, while the neck portion 9 at the opposite side receives a smaller force. That is to say, the neck portion 9 may avoid large deformation because of the protection of the broad portion 11. Under the cooperation of the first branch 7 and the second branch 8, the first strut 2 still keeps a good mechanical strength. Therefore, the slot 5 does not do harm to the overall mechanical properties of the blood vessel stent.

Fig. 5 is a schematic diagram of a second embodiment of an arched support piece on the absorbable blood vessel stent of the invention, which is roughly the same with the first embodiment. The differences lie in that: two longitudinally arranged slots 13 and 14 are arranged on the first strut 202, and divide the first strut 202 into two branches. Two longitudinally arranged slots similar to the slots 13 and 14 are also arranged on the second strut 302 which is connected with the first strut 202, and also divide the second strut 302 into two branches. Likewise, a neck portion 15 is arranged at the end where a branch is close to the peak segment, while another neck portion 17 is arranged at the opposite end of the other branch.

A bridge 16 exists between the slot 13 and the slot 14 to separate the slots 13 and 14. The width of the bridge 16 in the peripheral direction of the blood vessel stent 100 is the same as the widths of the neck portion 15 and the neck portion 17, and corresponds to the minimum width along the first support strut 202. Neck portions which are as wide as the neck portions 15 and 17 are also respectively arranged at the corresponding positions of the both branches on the second strut 302. The two slots are also separated by another bridge as wide as the bridge 16.

Using a bridge to connect two branches of the struts may increase the structural strength and stability of the support struts. The blood vessel stent 100 starts corrosion when implanted into the human body. The first strut 202, the neck portions 15 and 17 and the bridge 16 with the minimum width may break first, which causes the first strut 202 to completely break. Since the second strut 302 has a structure and size similar to the first strut 202, the two struts may simultaneously and completely break, so that the blood vessel stent 100 may break apart at this place in a shorter time.

Fig. 6 is a schematic diagram of configuration which is not part of the invention. This configuration includes an arched support piece on the absorbable blood vessel stent. The first strut 203 of the support piece is the same as the first strut 2 in the first embodiment of the support piece in shape and structure. The support piece also has a second strut 303, but the difference lies in that, no through slot is provided on the second strut 303. Compared with the first embodiment, the third embodiment may improve the overall stgth of the blood vessel stent, while the time from corrosion to disassembly of the blood vessel stent inside the human body is almost the same.

Fig. 7 is a schematic diagram of a fourth embodiment of an arched support piece on the absorbable blood vessel stent of the invention. The fourth embodiment of the support piece is similar to the first embodiment, and the differences lie in that the linear or straight line segment of the first strut 204 has a through slot 18 which is shorter than the above slot 13 and which is provided at a central position in the linear segment. It is preferred to set the length of the slot 18 to be one to three times of the width of the first strut 204. Another through slot similar to the slot 18 is also provided at the central position of the second strut 304.

The slot 18 divides the linear segment of the first strut 204 into two branches, that is, a first branch 19 and a second branch 20, and the two branches are the parts having the minimum width on the strut 204, that is, the neck portions. The first branch 19 and the second branch 20 will break first when the blood vessel stent is corroded, which will cause the first strut 204 to break completely. The second strut 304 has effects similar to the first strut 204, and also has a similar slots and branches. The two struts may simultaneously fully break, so that the blood vessel stent may disassemble at this place in a shorter time. Since the slot 18 herein is quite short and has a small effect on the structural strength of the strut, the broad portions in the first embodiment are unnecessary. In addition, the widths of the first branch 19 and the second branch 20 may be substantially uniform, the widths thereof may be equal, and are chosen from the range of 0.05 to 0.1 mm.

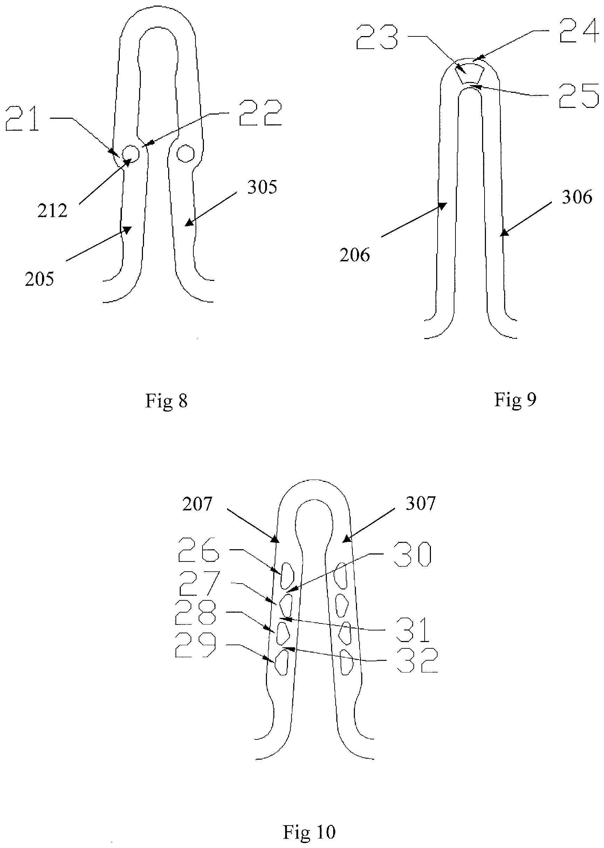

Fig. 8 is a schematic diagram of a configuration which is not part of the invention. The configuration of the support piece is similar to the fourth embodiment, and the differences lie in that the first strut 205 of the support piece has a slightly S-shaped curved segment at the central position thereof. A round or approximately round through hole 212 is arranged at this location. The second strut 305 is also slightly S-shaped, and also has such a through hole in the central position thereof. Thus the strength of the first strut 205 and the strut 305 is better than that of fourth embodiment.

Neck portions 21 and 22 are respectively arranged at both sides of the through hole 212. The widths of the neck portions 21 and 22 represent the minimum widths on the first support strut 205, such as 0.05 mm. The positions of the neck portions 21 and 22 will be first corroded and break, which causes the first strut 205 to break completely. Like the first strut 205, similar neck portions with the minimum widths are arranged at both sides of the through hole of the second strut 305, so that the blood vessel stent can break and disassemble in a shorter time.

Fig. 9 is a schematic diagram of another configuration which is not part of the invention. The configuration of the support piece is similar to the third embodiment, and the differences lie in that no through slots are provided on the first strut 206 and the second strut 306. An approximate fan-shaped through hole 23 is provided at the peak segment connecting the first strut 206 and the second strut 306. The through hole 23 divides the peak segment into two arched portions with small widths, that is, a first arched portion 24 at the outer side and a second arched portion 25 at the inner side.

The radial widths of the first arched portion 24 and the second arched portion 25 are basically equal, and the first arched portion 24 and the second arched portion 25 are the positions with the minimum widths of the whole support piece. These are neck portions, and the widths thereof are approximately 0.05 to 0.1 mm. Therefore, when the blood vessel stent is corroded inside the human body, the two arched portions 24 and 25 will break first, which causes the support piece to break completely at the wave peak, so that the blood vessel stent can disassemble at this place in a shorter time.

Adjacent arched support pieces are connected at a valley segment, so that similar structures with the through hole 23, the first arched portion 24, and the second arched portion 25 can be arranged at the valley segment. Since the blood vessel stent is corroded, the adjacent arched support pieces are disconnected at the valley segment in a shorter time.

If the blood vessel stent adopts a pattern structure of another form, then similar through slot, outer arched portion, and inner arched portion configurations may also be arranged at the curved segment of the support strut. When the blood vessel stent is expanded, the stress at the inner side is larger than the stress at the outer side of the peak or valley segment. Therefore, the second arched portion 25 at the inner side may be wider than the first arched portion 24 at the outer side, so as to enable the whole arched support piece to keep a good mechanical stability.

Fig. 10 is a schematic diagram of a comparative configuration which is not part of the invention. The configuration of the support piece is similar to the second embodiment, and the differences lie in that a truss structure composed of a plurality of bridge arms is arranged at the central portion of the first strut 207. The truss structure forms four through holes 26, 27, 28, and 29, while bridges 30, 31, and 32 are formed between the holes 26, 27, 28, and 29. The second strut 307 also has a similar truss structure at the central portion thereof. Several side bridges are provided along the outer sides of the central portion of the first strut 207 and the second strut 307. Each side bridge and bridge 30, 31, and 32 has a minimum width on the support strut, for example, the width may be chosen from 0.05 to 0.1 mm.

On the premise that the arched support piece has a higher structural strength and mechanical stability, the truss structures at the middle positions of the first strut 207 and the second strut 307 enable the blood vessel stent to use minimum amounts of material. Since the truss structure makes the metal amount reduced, it is easier for the blood vessel stent to be absorbed completely by the human body in a shorter time. The existence of each through hole increases the surface area of the blood vessel stent, the corrosion area is increased, and thus the disintegration of the blood vessel stent is accelerated.

In the absorbable blood vessel stent for treating blood vessel stenosis provided in the invention, the special structure thereof not only improve the properties of the iron blood vessel stent, but also can accelerate the corrosion and decomposition, compared to stents made of other absorbable materials. After blood vessel intimal cells wholly wrap the blood vessel stent, particular positions of this blood vessel stent can be corroded in a shorter time, so as to make the whole blood vessel stent break into several parts in the peripheral direction. In this way, the radial restraint of the stent for the blood vessel is removed, and thus normal increase of the blood vessel at this place will not be hindered.

Further, since the blood vessel stent is divided into several parts after disassembly, and is wrapped by intimal cells, the remnant structures are smaller, which helps the vascular tissues absorb the remnant parts of the blood vessel stent. The blood vessel stent of this structure may not weaken the overall mechanical property of the blood vessel stent, and the radial support force before disassembly can still meet the requirement of the diseased blood vessel. With the blood vessel stent of the invention, the time that the human body absorbs the stent is reduced, and meanwhile, the blood vessel stent is ensured to have a sufficient mechanical stability.

Compared with the prior art, the invention has the following advantages:
1. The absorbable blood vessel stent of the invention with a special structure can become corroded and decompose within a short time, which reduces the re-stenosis probability of the diseased blood vessel, contributes to continuous growth and expansion after the repair of the diseased blood vessel, and meets requirements of clinical use.
2. The structure of the absorbable blood vessel stent of the invention does not sacrifice the mechanical properties of the blood vessel stent. While promoting the corrosion and disintegration of the blood vessel stent, the blood vessel stent maintains the sufficient radial support force for the diseased blood vessel before disintegration.
3. The absorbable blood vessel stent of the invention may be made of an iron tubing, the wall thickness of the blood vessel stent is not increased compared with a common permanent blood vessel stent, and the stent may be delivered by adopting a balloon catheter which is commonly used for clinical application. Thus the cost of implementation of absorbable blood vessel stent is reduced, and the clinically applicable scope of the absorbable blood vessel stent is extended.

## Claims

1. An absorbable blood vessel stent comprising a proximal end, a distal end and a tubular pattern structure that is expandable and is formed between the proximal end and the distal end, wherein the pattern structure comprises a plurality of support struts (2, 3) and connection bridges (1), each connection bridge or support strut (2, 3) having a straight line segment, a U-shaped segment or a S-shaped segment, wherein at least one through slot is provided on first and second support struts (2, 3),
wherein the pattern structure comprises annular wavy bands with adjacent bands being coupled to one another by connection bridges (1), each annular wavy band comprising a plurality of arched support pieces connected adjacent one another at wave peak segments (4) and at wave valley segments, such that a first support strut (2) and a second support strut (3) are connected by a wave peak segment (4),
**characterized in that** first and second through slots (5, 6) are provided in a central portion of the respective first and second struts (2, 3), where either end of the first and second slots (5, 6) are close to positions of the wave peak segment (4) and the wave valley segment,
wherein each of the first slot (5) and the second slot (6) divides the first strut (2) and respectively the second strut (3) into a first branch (7) and a second branch (8) and a first neck portion (9) is formed at a position where the first branch (7) is close to the wave peak segment (4), the first neck portion (9) being the position of minimum width of the first branch (7), and wherein a first broad portion (10) is formed at the other end of the first branch (7) which is the position of maximum width on the first branch (7) and wherein the width of the first branch (7) extending from the first neck portion (9) to the first broad portion (10) gradually changes, and
wherein the second branch (8) has a second broad portion (11) at a position opposite the first neck portion (9), the second broad portion (11) being the position of maximum width on the second branch (8), and wherein a second neck portion (12) defining the position of minimum width on the second branch (8) is formed at a position opposite the first broad portion (10) of the first branch (7) and wherein the width of the second branch (8) changes gradually from the second neck portion (12) to the second broad portion (11).

2. The absorbable blood vessel stent according to claim 1, wherein each of the first slot (5) and the second slot (6) is formed of two longitudinally arranged slots (13, 14) provided on the straight line segment of the first support strut (2) or respectively the second support strut (3) which run parallel to the straight line segment, wherein the two longitudinally arranged slots (13, 14) and the straight line segment together form two branches (7, 8), the branches having the minimum width along the first support strut (2) and respectively the second support strut (3) and wherein the two longitudinally arranged slots (13, 14) are arrayed along the first support strut (2) and respectively the second support strut (3) with a bridge (16) arranged between the two adjacent slots, the bridge having the minimum width on the support strut (2) and respectively the support strut (3).

3. The absorbable blood vessel stent according to claim 1 or 2, wherein the first support strut (2) and the second support strut (3) are symmetric with one another.

4. The absorbable blood vessel stent according to claim 1, wherein the minimum width along the support strut is 0.05 to 0.1 mm.

5. The absorbable blood vessel stent according to claim 1, wherein the blood vessel stent is made of iron or iron alloy material.

## Patentansprüche

1. Resorbierbarer Blutgefäßstent, der ein proximales Ende, ein distales Ende und eine Struktur mit röhrenförmigem Muster umfasst, die dehnungsfähig ist und zwischen dem proximalen Ende und dem distalen Ende gebildet ist, wobei die Musterstruktur mehrere Stützstreben (2, 3) und Verbindungsbrücken (1) umfasst, wobei jede Verbindungsbrücke bzw. Stützstrebe (2, 3) einen geradlinigen Abschnitt, einen U-förmigen Abschnitt oder einen S-förmigen Abschnitt aufweist, wobei an der ersten und der zweiten Stützstrebe (2, 3) mindestens ein Durchgangsschlitz vorgesehen ist,
wobei die Musterstruktur ringförmige gewellte Bänder umfasst, wobei benachbarte Bänder über Verbindungsbrücken (1) miteinander verbunden sind, wobei jedes ringförmige gewellte Band mehrere bogenförmige Trägerstücke umfasst, die benachbart zueinander an Wellenkammabschnitten (4) und an Wellentalabschnitten so verbunden sind, dass eine erste Stützstrebe (2) und eine zweite Stützstrebe (3) über einen Wellenkammabschnitt (4) verbunden sind,
**dadurch gekennzeichnet, dass** in einem mittleren Bereich der ersten bzw. der zweiten Strebe (2, 3) ein erster bzw. ein zweiter Durchgangsschlitz (5, 6) vorgesehen ist, wobei beide Enden des ersten und des zweiten Schlitzes (5, 6) nahe an Positionen des Wellenkammabschnitts (4) bzw. des Wellentalabschnitts liegen,
wobei der erste Schlitz (5) und der zweite Schlitz (6) jeweils die erste Strebe (2) bzw. die zweite Strebe (3) in einen ersten Zweig (7) und einen zweiten Zweig (8) teilt und ein erster Halsbereich (9) an einer Stelle gebildet ist, an der sich der erste Zweig (7) nahe an dem Wellenkammabschnitt (4) befindet, wobei der erste Halsbereich (9) die Stelle minimaler Breite des ersten Zweiges (7) ist, und wobei am anderen Ende des ersten Zweiges (7) ein erster breiter Bereich (10) gebildet ist, der die Stelle maximaler Breite am ersten Zweig (7) ist, und wobei sich die Breite des ersten Zweiges (7), der sich vom ersten Halsbereich (9) zum ersten breiten Bereich (10) erstreckt, allmählich ändert, und
wobei der zweite Zweig (8) an einer dem ersten Halsbereich (9) gegenüberliegenden Stelle einen zweiten breiten Bereich (11) aufweist, wobei der zweite breite Bereich (11) die Stelle maximaler Breite am zweiten Zweig (8) ist, und wobei ein zweiter Halsbereich (12), der die Stelle minimaler Breite am zweiten Zweig (8) definiert, an einer dem ersten breiten Bereich (10) des ersten Zweiges (7) gegenüberliegenden Stelle gebildet ist und wobei sich die Breite des zweiten Zweiges (8) vom zweiten Halsbereich (12) zum zweiten breiten Bereich (11) allmählich ändert.

2. Resorbierbarer Blutgefäßstent nach Anspruch 1, bei dem der erste Schlitz (5) und der zweite Schlitz (6) jeweils aus zwei längs angeordneten Schlitzen (13, 14) gebildet sind, die an dem geradlinigen Abschnitt der ersten Stützstrebe (2) bzw. der zweiten Stützstrebe (3) vorgesehen sind und parallel zu dem geradlinigen Abschnitt verlaufen, wobei die beiden längs angeordneten Schlitze (13, 14) und der geradlinige Abschnitt zusammen zwei Zweige (7, 8) bilden, wobei die Zweige die minimale Breite längs der ersten Stützstrebe (2) bzw. der zweiten Stützstrebe (3) aufweisen und wobei die beiden längs angeordneten Schlitze (13, 14) längs der ersten Stützstrebe (2) bzw. der zweiten Stützstrebe (3) angeordnet sind, wobei eine Brücke (16) zwischen den beiden benachbarten Schlitzen angeordnet ist, wobei die Brücke die minimale Breite an der Stützstrebe (2) bzw. der Stützstrebe (3) aufweist.

3. Resorbierbarer Blutgefäßstent nach Anspruch 1 oder 2, bei dem die erste Stützstrebe (2) und die zweite Stützstrebe (3) zueinander symmetrisch sind.

4. Resorbierbarer Blutgefäßstent nach Anspruch 1, bei dem die minimale Breite längs der Stützstrebe 0,05 bis 0,1 mm beträgt.

5. Resorbierbarer Blutgefäßstent nach Anspruch 1, wobei der Blutgefäßstent aus Eisen oder einem Eisenlegierungsmaterial besteht.

## Revendications

1. Stent absorbable pour vaisseau sanguin, présentant une extrémité proximale, une extrémité distale et une structure à motif tubulaire qui est extensible et est formée entre l'extrémité proximale et l'extrémité distale, la structure à motif comprenant une pluralité de barrettes de support (2, 3) et des ponts de raccordement (1), chaque pont de raccordement ou barrette de support (2, 3) présentant un segment rectiligne, un segment en forme de U ou un segment en forme de S, au moins une fente de passage étant prévue sur la première et la deuxième barrette de support (2, 3),
la structure à motif comprenant des bandes ondulées annulaires, des bandes adjacentes étant reliées les unes aux autres par des ponts de raccordement (1), chaque bande ondulée annulaire comprenant une pluralité de pièces de support arquées reliées les unes à côté des autres au niveau de segments en crête de vague (4) et de segments en creux de vague, de sorte qu'une première barrette de support (2) et une deuxième barrette de support (3) sont reliées par un segment en crête de vague (4),
**caractérisé en ce qu'**une première et une deuxième fente de passage (5, 6) sont respectivement prévues dans un tronçon médian de la première et la deuxième barrette (2, 3), chaque extrémité de la première et de la deuxième fente (5, 6) étant proche de positions du segment en crête de vague (4) et du segment en creux de vague,
la première fente (5) et la deuxième fente (6) divisant respectivement la première barrette (2) et la deuxième barrette (3) en une première branche (7) et une deuxième branche (8), et un premier tronçon de gorge (9) étant formé à une position où la première branche (7) est proche du segment en crête de vague (4), le premier tronçon de gorge (9) étant la position à largeur minimale de la première branche (7), et un premier tronçon large (10) étant formé à l'autre extrémité de la première branche (7) qui est la position à largeur maximale sur la première branche (7), et la largeur de la première branche (7) qui s'étend du premier tronçon de gorge (9) au premier tronçon large (10) changeant progressivement, et
la deuxième branche (8) présentant un deuxième tronçon large (11) à une position opposée au premier tronçon de gorge (9), le deuxième tronçon large (11) étant la position à largeur maximale sur la deuxième branche (8), et un deuxième tronçon de gorge (12) qui définit la position à largeur minimale sur la deuxième branche (8) étant formé à une position opposée au premier tronçon large (10) de la première branche (7), et la largeur de la deuxième branche (8) changeant progressivement du deuxième tronçon de gorge (12) au deuxième tronçon large (11).

2. Stent absorbable pour vaisseau sanguin selon la revendication 1, la première fente (5) et la deuxième fente (6) étant réalisées à partir de deux fentes (13, 14) agencées longitudinalement et respectivement prévues sur le segment rectiligne de la première barrette de support (2) ou de la deuxième barrette de support (3) et s'étendant de manière parallèle au segment rectiligne, les deux fentes (13, 14) agencées longitudinalement et le segment rectiligne formant ensemble deux branches (7, 8), les branches présentant respectivement la largeur minimale le long de la première barrette de support (2) et de la deuxième barrette de support (3), et les deux fentes (13, 14) agencées longitudinalement étant respectivement agencées la long de la première barrette de support (2) et de la deuxième barrette de support (3), un pont (16) étant agencé entre les deux fentes adjacentes, le pont présentant respectivement la largeur minimale sur la barrette de support (2) et la barrette de support (3).

3. Stent absorbable pour vaisseau sanguin selon la revendication 1 ou 2, la première barrette de support (2) et la deuxième barrette de support (3) étant symétriques l'une par rapport à l'autre.

4. Stent absorbable pour vaisseau sanguin selon la revendication 1, la largeur minimale le long de la barrette de support étant comprise entre 0,05 et 0,1 mm.

5. Stent absorbable pour vaisseau sanguin selon la revendication 1, le stent pour vaisseau sanguin étant réalisé en fer ou en un matériau d'alliage de fer.
